Europäisches Patentamt

European Patent Office

Office européen des brevets

(11) Veröffentlichungsnummer: **0 322 813**
**A2**

(12) **EUROPÄISCHE PATENTANMELDUNG**

(21) Anmeldenummer: 88121626.1

(22) Anmeldetag: 23.12.88

(51) Int. Cl.4: **G01N 33/537 , G01N 33/543**

(30) Priorität: 24.12.87 DE 3744067
11.02.88 DE 3804244

(43) Veröffentlichungstag der Anmeldung:
05.07.89 Patentblatt 89/27

(84) Benannte Vertragsstaaten:
**AT BE CH DE ES FR GB GR IT LI LU NL SE**

(71) Anmelder: **BOEHRINGER MANNHEIM GmbH**
**Sandhofer Strasse 112-132**
**D-6800 Mannheim Waldhof(DE)**

(72) Erfinder: **Rüdinger, Wolfgang, Dr.med. Dr.-Ing.**
**Balzenbacher Strasse 66**
**D-6943 Birkenau(DE)**
Erfinder: **Schmitt,Urban**
**Waldstrasse 36**
**D-8125 Oberhausen(DE)**
Erfinder: **Maier,Josef**
**Schmädlstrasse 15**
**D-8120 Weilheim(DE)**

(74) Vertreter: **Huber, Bernhard, Dipl.-Chem. et al**
**Möhlstrasse 22 Postfach 860 820**
**D-8000 München 86(DE)**

(54) **Verfahren zur Bestimmung einer immunologisch aktiven Substanz.**

(57) Zur Bestimmung einer immunologisch aktiven Substanz nach dem Prinzip des Immunoassays durch Inkubation mit mindestens 2 verschiedenen, in flüssiger Phase gelöst vorliegenden Rezeptoren $R_1$ und $R_2$, von denen mindestens einer mit der immunologisch aktiven Substanz bindefähig ist, wobei der zweite Rezeptor $R_2$ eine Markierung trägt, Abtrennung des sich bildenden Komplexes aus der Lösung durch Bindung an eine feste Phase und Messung der Markierung in einer der Phasen, verwendet man einen ersten Rezeptor $R_1$, der an eine erste spezifisch bindefähige Substanz $S_1$ gebunden ist, inkubiert die zu bestimmende immunologisch aktive Substanz mit den Rezeptoren in Gegenwart einer Matrix, an die eine zweite spezifisch bindefähige Substanz $S_2$ gebunden ist und gibt nach der Inkubation zur Immobilisierung des Komplexes eine Komponente zu, die mindestens je eine spezifische Bindungsstelle für $S_1$ und $S_2$ aufweist und trennt danach die Phasen.

FIG.1

EP 0 322 813 A2

## Verfahren zur Bestimmung einer immunologisch aktiven Substanz

Die Erfindung betrifft ein Verfahren zur Bestimmung einer immunologisch aktiven Substanz nach dem Prinzip des Immunoassays durch Inkubation mit mindestens zwei in flüssiger Phase gelöst vorliegenden Rezeptoren $R_1$ und $R_2$, von denen mindestens einer mit der immunologisch aktiven Substanz bindefähig ist, wobei der zweite Rezeptor $R_2$ eine Markierung trägt, Abtrennung des sich bildenden Komplexes aus der Lösung durch Bindung an eine feste Phase und Messung der Markierung in einer der Phasen sowie ein dazu geeignetes Reagenz.

Die Entwicklung von Bestimmungsverfahren nach dem Prinzip des Immunoassays hat, ausgehend vom Radioimmunoassay zu einer entscheidenden Verbesserung der Empfindlichkeit und Selektivität der Bestimmung von immunologisch aktiven Substanzen geführt. Insbesondere in den klinisch-chemischen Laboratorien haben Bestimmungen nach dem Prinzip des Immunoassays eine große Bedeutung erlangt. Durch die Weiterentwicklung dieser Methoden wurden einerseits die zur Verfügung stehenden Markierungsmöglichkeiten vergrößert, wobei neben der radioaktiven Markierung vor allem die Enzymmarkierung und auch die Markierung mit fluoreszierenden Substanzen Bedeutung erlangt hat und andererseits auch die Entwicklung der Umsetzungstechniken erheblich differenziert. Dabei wurden sowohl in homogener als auch in heterogener Phase ablaufende Tests entwickelt. Ein bei der Durchführung dieser Verfahren auftretendes Problem besteht darin, das Konjugat aus zu bestimmender Substanz und markiertem Antikörper von ungebundenem markiertem Antikörper abzutrennen, um aus der Messung entweder der gebundenen oder der ungebundenen Markierung auf den Anteil an zu bestimmender Substanz rückzuschließen. Es hat sich nun als vorteilhaft erwiesen, einen der Rezeptoren an eine Festphase zu binden, da dies die Trennung von gebundenen und nicht gebunden vorliegenden Reaktionspartnern erleichtert. Hierzu wurden sehr viele Varianten entwickelt, beispielsweise auf Basis eines Sandwichassays, bei dem die zu bestimmende Substanz an einen markierten Antikörper und einen an eine Festphase gebundenen Antikörper gebunden wird, wobei das an der Festphase gebundene Konjugat aus Antikörper, zu bestimmender Substanz und markiertem Antikörper leicht von der restlichen Flüssigkeit abgetrennt werden kann. Nachteil dieser Verfahren ist es jedoch, daß die Reaktion von zu bestimmender Substanz bzw. der Komplex aus zu bestimmender Substanz und markiertem Antikörper mit dem immobilisierten Rezeptor in heterogener Phase abläuft. Dies führt insbesondere zu langen Reaktionszeiten und zu Verschleppungs- und Verdünnungsproblemen.

Ein weiterer Nachteil dieser bekannten Verfahren ist es, daß für jeden Test eine spezifische Festphase zur Verfügung gestellt werden muß, d.h. ein Trägermaterial, an dem der für die spezifische Reaktion benötigte Antikörper bzw. Rezeptor gebunden ist. Dies ist sehr aufwendig.

Es war daher Aufgabe der vorliegenden Erfindung, ein Verfahren zu schaffen, bei dem die Umsetzung der zu bestimmenden Substanz mit den direkt bindenden Rezeptoren in homogener Phase verläuft und bei dem eine Universalmatrix verwendet werden kann, die für viele verschiedene Testverfahren geeignet ist.

Diese Aufgabe wird gelöst durch ein Verfahren zur Bestimmung einer immunologisch aktiven Substanz nach dem Prinzip des Immunoassays durch Inkubation mit mindestens zwei in flüssiger Phase gelöst vorliegenden Rezeptoren $R_1$ und $R_2$, von denen mindestens einer mit der immunologisch aktiven Substanz bindefähig ist, wobei der zweite Rezeptor $R_2$ eine Markierung trägt, Abtrennung des sich bildenden Komplexes aus der Lösung durch Bindung an eine feste Phase und Messung der Markierung in einer der Phasen, das dadurch gekennzeichnet ist, daß man einen ersten Rezeptor $R_1$ verwendet, der an eine erste spezifisch bindefähige Substanz $S_1$ gebunden ist, die zu bestimmende immunologisch aktive Substanz mit den Rezeptoren inkubiert in Gegenwart einer Matrix, an die eine zweite spezifisch bindefähige Substanz $S_2$ gebunden ist und daß man nach der Inkubation zur Immobilisierung des Komplexes eine Komponente zugibt, die mindestens je eine spezifische Bindungsstelle für $S_1$ und $S_2$ aufweist und danach die Phasen trennt.

Dieses Verfahren ist sehr vorteilhaft, da die Umsetzung der immunologisch aktiven Substanz mit den Rezeptoren in homogener Phase stattfindet, was einerseits zu einer Erhöhung der Empfindlichkeit und andererseits zu einer signifikanten Verkürzung der Inkubationszeiten führt. Darüberhinaus kann die für das Verfahren verwendete Universalmatrix unabhängig von der Art der zu bestimmenden Substanz in vielen verschiedenen Verfahren eingesetzt worden. Dies vereinfacht die Herstellung und Anwendung der Matrix.

Das erfindungsgemäße Verfahren dient zur Bestimmung immunologisch aktiver Substanzen. Unter immunologisch aktiven Substanzen werden hier Moleküle verstanden, die eine immunologische Reaktion eingehen können. Insbesondere sind dies Antigene, Haptene und Antikörper.

Die die zu bestimmende immunologisch aktive Substanz enthaltende Lösung wird dazu mit mindestens

2

zwei Rezeptoren $R_1$ und $R_2$ umgesetzt. Als Rezeptoren werden im Rahmen der Erfindung spezifisch bindefähige komplette Antikörper, die polyklonal oder monoklonal sein können, deren Antikörperfragmente oder Konjugate von Antikörpern oder Antikörperfragmenten mit Haptenen oder Antigenen, sowie Haptene und mono- oder polyvalente Antigene verstanden. Als polyvalentes Antigen wird ein Antigen bezeichnet, das mehrere nicht notwendig gleiche Epitope besitzt.

Mindestens einer der erfindungsgemäß eingesetzten Rezeptoren ist bindefähig mit der zu bestimmenden immunologisch aktiven Substanz unter Bildung eines Komplexes. Ist die zu bestimmende Substanz ein Antigen, so ist bevorzugt der erste Rezeptor $R_1$ ein kompletter Antikörper und der zweite Rezeptor $R_2$ ein Antikörper oder Antikörperfragment mit einer Markierung. Ist die zu bestimmende Substanz ein Antikörper, so kann $R_1$ z.B. ein IgM-Antikörper sein und $R_2$ das markierte, spezifische Antigen sein. Die Markierung kann ein Enzym, eine radioaktive Substanz, ein Isotop, eine fluoreszierende oder chemilumineszierende Substanz sein, deren Bestimmung nach einer der dem Fachmann wohlbekannten Methoden durchgeführt wird. So kann beispielsweise, wenn die Markierung mit einem Enzym wie Peroxidase oder $\beta$-Galactosidase erfolgt, die Aktivität dieses Enzyms in der hierfür allgemein bekannten Weise gemessen werden, indem man ein bekanntes Nachweisreagenz für dieses Markierungsenzym, z.B. ein Farbreagenz, zusetzt und entweder in Anwesenheit der festen Phase oder nach Trennung mißt. Die gemessene Enzymaktivität ist dann ein Maß für die Menge an zu bestimmender Substanz.

Der erste Rezeptor $R_1$ ist an eine erste spezifisch bindefähige Substanz $S_1$ gebunden. Als spezifisch bindefähige Substanz wird dabei ein Partner eines spezifisch bindefähigen Paares verwendet. Als Beispiele können genannt werden die Paare Antigen-Antikörper; Hapten-Antikörper; Biotin-Antibiotin-Antikörper; Biotin-Avidin; Biotin-Streptavidin; Protein A-Immun-$\gamma$-Globulin. Dem Fachmann sind solche Bindungspaare bekannt. Bevorzugt wird als $S_1$ ein Hapten verwendet, wie beispielsweise Digoxin, p-Nitrophenol, Saponin, FITC oder Biotin.Die Bindung der spezifisch bindefähigen Substanz an den Rezeptor $R_1$ erfolgt in an sich bekannter Weise.

Weiterhin wird an eine Matrix ebenfalls eine spezifisch bindefähige Substanz $S_2$ gebunden. Auch hier kommen wieder Partner eines spezifisch bindefähigen Paares in Betracht. Die spezifisch bindefähige Substanz $S_2$ kann dieselbe sein wie $S_1$ oder aber verschieden davon. Die spezifisch bindefähige Substanz $S_2$ wird an die Matrix in an sich bekannter Weise gebunden. Hierzu sind, abhängig von der Art der verwendeten spezifisch bindefähigen Substanz verschiedene Verfahren möglich. Besitzt $S_2$ funktionelle Gruppen, so kann es beispielsweise direkt oder über einen Spacer an die Matrix gebunden werden. Weiterhin ist es ebenfalls möglich, $S_2$ an ein Protein zu binden, das wiederum an der Matrix adsorbiert wird. Je nach den Gegebenheiten wird die jeweils günstigste Art der Bindung ausgewählt, so, daß die spezifische Bindungsstelle frei zugänglich ist, um eine Bindung mit dem zweiten Partner des Bindepaares zu ermöglichen.

Als Matrix, an die $S_2$ gebunden wird, sind sowohl Polymermaterialien als auch cellulosehaltige Materialien oder Glas geeignet. Als besonders geeignet haben sich Polystyrol, Polymethacrylat, Teflon, Polyamid, Copolymere aus Styrol und Acrylnitril, Glas- und Celluloseprodukt erwiesen. Die Matrix kann in beliebiger Form vorliegen, z.B. als Röhrchen, Mikrotiterplatten, Kugeln, Film, Pulver, Körnchen oder Faservlies.

$S_2$ kann in an sich bekannter Weise an die Festphase gebunden werden.

In einer bevorzugten Ausführungsform des erfindungsgemäßen Verfahrens liegt $S_2$ in hochmolekularer Form vor. Dazu kann $S_2$ entweder homogen vernetzt sein oder aber mit anderen, in bezug auf die immunologische Aktivität inerten Substanzen, gebunden sein. Geeignet sind beispielsweise nach einem in der Patentanmeldung P 36 40 412 beschriebenen Verfahren erhaltene Festphasen. Dazu wird $S_2$ an ein hydrophobes Protein mit einem Molekulargewicht über etwa 500.000 gebunden, das wiederum an der hydrophoben Matrix adsorbiert ist. Das Protein ist dabei hydrophober als $S_2$. Zur Auswahl von geeigneten löslichen Proteinen muß das Molekulargewicht sowie die Hydrophobizität im Vergleich zu dem entsprechenden Wert für $S_2$ ermittelt werden. Das Molekulargewicht wird nach den dem Fachmann bekannten Methoden ermittelt.

Ein Vergleich der Hydrophobizität zwischen löslichem Protein und $S_2$ kann ebenfalls nach den dem Fachmann geläufigen Methoden erfolgen. Geeignete Methoden sind beispielsweise der Vergleich

-der Fluoreszenzlöschung nach Bindung an Farbstoffe (Biochem. Biophys. Acta 624, (1980), 13-20);
- des Elutionsverhaltens bei der hydrophoben Chromatographie (Biochem. Biophys. Acta, 576 (1979), 269-279);
- der Oberflächenspannung (Biochem. Biophys. Acta 670 (1981), 64-73);
- der Retentionszeiten bei Hydrophobic Interaction Chromatography (HIC) (Angew. Chemie 98 (1986) 530-548, J. Chromat. 296 (1984) 107-114, Anal. Biochem. 137, (1984) 464-472).

3

Ein Vergleich der Hydrophobizität von geeigneten Substanzen findet sich in Sep. Sci. Technol. 14, 305-317 (1979). Danach steigt die Hydrophobizität z.B. in folgender Reihe an:

$\alpha_2$-Makroglobulin (MG 820.000),

Rinderserumalbumin/Humanserumalbumin (MG ca. 70.000),

Eialbumin,

$\alpha_2$HS-Glykoprotein (MG ca. 49.000),

$\beta 1c/\beta 1a$-Globulin,

Immunglobulin (MG ca. 150.000),

Transferrin (MG ca. 90.000).

Wird also als $S_2$ ein Immunglobulin verwendet, sind beispielsweise Humanserumalbumin oder $\alpha_2$HS-Glykoprotein als lösliche Proteine ohne weitere Vorbehandlung nicht geeignet. Beide Proteine müssen hier sowohl einer Hydrophobisierung als auch einer Erhöhung des Molekulargewichts unterworfen werden. Bei Transferrin genügt in diesem Fall eine Vernetzung, bei $\alpha_2$-Makroglobulin ist eine Hydrophobisierung ausreichend.

Proteine, die zur Kupplung mit Immunglobulin als $S_2$ ohne Vorbehandlung geeignet sind, sind beispielsweise $\beta$-Lipoproteine (MG ca. 3,2 Mio) oder $\alpha_2$-Lipoprotein (MG ca. 5 bis 20 Mio).

Die Hydrophobisierung kann beispielsweise durch Anwendung von Hitze, Behandlung mit Säuren, denaturierenden Agentien und/oder chaotropen Ionen und/oder durch chemische Kupplung mit einer hydrophoben Verbindung erfolgen.

Die Erhöhung des Molekulargewichts kann beispielsweise durch Anwendung von Hitze, Behandlung mit Säuren, denaturierenden Agentien und/oder chaotropen Ionen und/oder durch Vernetzung mit einem bi- oder polyfunktionellen Proteinreagenz erfolgen.

Die Behandlung wird so lange durchgeführt, bis ein Proteinpolymeres mit einem Molekulargewicht von 500.000 oder mehr erhalten wird. Besonders bevorzugt ist es, ein Proteinpolymeres mit einem Molekulargewicht von 500.000 bis 20.000.000 zu verwenden.

Die Hydrophobisierung kann dann, wenn auch eine Vernetzung erfolgen soll, vor, während oder nach der Vernetzung, jedoch nicht in Gegenwart von $S_2$ vorgenommen werden.

Zur Hydrophobisierung durch Erhitzen werden üblicherweise Temperaturen von 40 bis 95 °C in einem Zeitraum von 1 Minute bis 10 Stunden angewendet, wie beispielsweise in Biochem. Biophys. Acta 624 - (1980) 13-20 beschrieben.

Die Behandlung mit Säuren kann beispielsweise mit Essigsäure, Propionsäure, Milchsäure oder Salzsäure erfolgen. Übliche Konzentrationen sind 1 bis 100 mmol/l bei Einwirkzeiten von 10 Minuten bis 16 Stunden.

Geeignete chaotrope Ionen sind beispielsweise Thiocyanate, Jodide, Fluoride, Bromide, Perchlorate und Sulfate. Geeignete denaturierende Agentien sind beispielsweise Guanidinhydrochlorid oder Harnstoff. Üblicherweise werden hier Konzentrationen von 10 mmol/l bis 6 Mol/l angewendet.

Zur Derivatisierung mit hydrophoben Verbindungen werden vorzugsweise lösliche Fettsäuren, Lipoide in nieder-oder hochmolekularer Form sowie synthetische Polymere, wie Polypropylenglykol oder lösliche Copolymere von Polystyrol eingesetzt. Die Derivatisierung erfolgt nach den dem Fachmann geläufigen Methoden.

Die Vernetzung über bi- oder polyfunktionelle Verbindungen wird mit an sich bekannten Proteinbindungsreagenzien durchgeführt. Dies sind Verbindungen, die mindestens zwei funktionelle Gruppen tragen, die gleich oder verschieden sein können und die über diese funktionellen Gruppen mit funktionellen Gruppen von Proteinen reagieren können. Bevorzugt werden Verbindungen verwendet, die aus einer Alkylkette bestehen, an deren Enden sich Succinimid-, Maleinimid- und/oder Aldehydgruppen befinden.

Das Protein wird mit der bi- oder polyfunktionellen Verbindung in an sich bekannter Weise vernetzt, indem das lösliche Protein und die bi- oder polyfunktionelle Verbindung umgesetzt werden.

Bevorzugt werden zur Hydrophobisierung und/oder Vernetzung Proteine mit einem Molekulargewicht von 10.000 bis 700.000 verwendet. Besonders bevorzugt wird Serumalbumin, Lipase oder Immun-$\gamma$-Globulin eingesetzt.

An das Protein wird in an sich bekannter Weise $S_2$ gekuppelt. Geeignete Kupplungsmethoden sind z.B. in Ishikawa, J. Immunoassay,4, 209-327 (1983) beschrieben.

Das Protein, an das $S_2$ gebunden ist, wird dann an der als Festphase dienenden Matrix adsorbiert. Die adsorptive Bindung an die Festphase erfolgt über starke und schwache Wechselwirkungen, hydrophobe Kräfte, Dipol-Dipol- oder Ionen-Dipol-Interaktionen.

Bei der Durchführung des erfindungsgemäßen Verfahrens werden in einem geeigneten Reaktionsgefäß, wie beispielsweise einem Röhrchen oder einer Mikrotiterplatte, der erste Rezeptor $R_1$, der an die erste

4

spezifisch bindefähige Substanz $S_1$ gebunden ist, der zweite Rezeptor $R_2$, der die Markierung trägt und die Probe in Gegenwart einer Matrix, an die die zweite spezifisch bindefähige Substanz $S_2$ gebunden ist, inkubiert. Dabei erfolgt die Reaktion von $R_1$ und $R_2$ mit der zu bestimmenden immunologisch aktiven Substanz in homogener Phase, was die Inkubationszeit gegenüber den bisher bekannten Verfahren sehr stark verkürzt. So kann mit dem erfindungsgemäßen Verfahren eine ausreichende Inkubationszeit im Bereich von 10 Minuten, gegenüber den bisher bekannten Inkubationszeiten von ca. 4 Stunden bei Raumtemperatur erreicht werden.

Nach der Inkubation wird dann eine Komponente zugegeben, die zwei spezifische Bindungsstellen für $S_1$ und $S_2$ aufweist, wodurch die Immobilisierung des aus $R_1$, $R_2$ und der zu bestimmenden immunologisch aktiven Substanz gebildeten Konjugates führt. Diese Komponente muß also mindestens zwei spezifische Bindungsstellen aufweisen.

Diese Komponente kann ein Molekül sein, das zwei oder mehr Bindungsstellen für $S_1$ bzw. $S_2$ aufweist, sie kann ebenso auch ein Konjugat aus zwei verschiedenen Molekülen sein, die jeweils eine spezifische Bindungsstelle für $S_1$ bzw. $S_2$ aufweisen. Weiterhin ist es möglich, die Komponente in vernetzter Form einzusetzen, d.h. also Moleküle mit Bindungsstellen für $S_1$ und $S_2$, die miteinander vernetzt sind.

Bevorzugt sind erfindungsgemäß $S_1$ und $S_2$ identisch, so daß dann auch die Komponente zwei identische Bindungsstellen aufweist. Besonders bevorzugt wird als $S_1$ und $S_2$ jeweils Biotin eingesetzt. Als Komponente wird dann Avidin oder Streptavidin verwendet, die jeweils vier Bindungsstellen für Biotin aufweisen.

Durch Zugabe der Komponente zu der Lösung wird das gebildete Konjugat aus $R_1$ und/oder $R_2$ und zu bestimmender immunologisch aktiver Substanz durch die Bindung der Komponente mit $S_1$ und die Bindung der Komponente mit $S_2$ an der Festphase fixiert. Die Phasen können dann leicht getrennt werden, indem die Flüssigkeit entfernt wird. Nach Trennung der Phasen kann dann die Markierung in einer der beiden Phasen in an sich bekannter Weise bestimmt werden und ist dann ein Maß für die Menge an vorhandener immunologisch aktiver Substanz.

Das erfindungsgemäße Bestimmungsverfahren ist für alle bekannten Variationen von Immunoassays geeignet, bei denen mindestens zwei Rezeptoren eingesetzt werden. Es ist insbesondere geeignet für Verfahren nach dem Prinzip des Sandwichassays und für kompetitive Assays.

Für ein Verfahren nach dem Prinzip des Sandwichassays werden an einer Festphase spezifisch bindende Substanzen $S_2$ fixiert. In Gegenwart der Festphase wird dann die Probelösung mit einem Rezeptor, der an eine spezifisch bindende Substanz $S_1$ gebunden ist und mit der zu bestimmenden Substanz spezifisch bindefähig ist sowie einem Rezeptor, der eine Markierung trägt und ebenfalls mit der zu bestimmenden Substanz spezifisch bindefähig ist, inkubiert. Die Zugabe der beiden Rezeptoren kann gleichzeitig oder nacheinander erfolgen. Der sich bildende Komplex aus markiertem Rezeptor, zu bestimmender Substanz und an $S_1$ gebundenem Rezeptor wird dann an der Festphase immobilisiert durch Zugabe einer Komponente, die Bindungsstellen für $S_1$ und $S_2$ aufweist. Diese zugegebene Komponente bindet dann sowohl an die Festphase, als auch an das Konjugat. Nach Trennung der flüssigen von der festen Phase kann dann die Markierung in einer der beiden Phasen in an sich bekannter Weise bestimmt werden. Als Beispiel für eine derartige Verfahrensweise kann an die Festphase Biotin gebunden sein. Als Rezeptoren können dann zwei gegen die zu bestimmende Substanz gerichtete Rezeptoren verwendet werden, von denen einer in üblicher Weise markiert ist, während der andere biotinyliert ist. Durch Zugabe von Streptavidin oder Avidin oder aber auch Anti-Biotin-Antikörper erfolgt dann die Immobilisierung.

Ebenso geeignet ist das erfindungsgemäße Verfahren für Bestimmungen nach dem Prinzip des kompetitiven Assays. Dabei wird wiederum an eine Festphase eine spezifisch bindefähige Substanz $S_2$ gebunden. In einer Ausführungsform wird dann die Probelösung, die die zu bestimmende Substanz enthält, mit einem markierten, mit der zu bestimmenden Substanz spezifisch bindenden Rezeptor und einer Verbindung, die der zu bestimmenden Substanz entspricht und an die $S_1$ gebunden ist, inkubiert. Die zu bestimmende Substanz und die an $S_1$ gebundene Verbindung konkurrieren dann um den markierten spezifisch bindefähigen Rezeptor. Nach Zugabe der $S_1$ und $S_2$ bindenden Komponente werden dann die Konjugate aus markiertem Rezeptor und an $S_1$ gebundener Verbindung immobilisiert. Die Menge an gebundener Markierung ist dann ein Maß für die in der Probe vorliegende Substanz. Je größer die Menge an zu bestimmender Substanz ist, desto mehr markierte Rezeptoren reagieren mit der zu bestimmenden Substanz und desto weniger Konjugate aus an $S_1$ gebundener Verbindung und markiertem Rezeptor bilden sich, so daß an der Festphase nach Zugabe der Komponente um so weniger markierter Rezeptor nachgewiesen werden kann. Das Umgekehrte gilt dann, wenn die zu bestimmende Substanz in der Lösung in geringer Menge vorhanden ist. Eine weitere Variante des kompetitiven Assays besteht darin, daß man die Probelösung, die die zu bestimmende Substanz enthält, mit einem mit der zu bestimmenden Substanz spezifisch bindefähigen Rezeptor, an den $S_1$ gebunden ist, sowie einer Verbindung, die der zu bestimmenden Substanz entspricht und eine Markierung trägt, inkubiert. Hierbei konkurrieren die zu bestimmende Substanz sowie die markierte Verbindung um den an $S_1$ gebundenen Rezeptor. Nach Zugabe der $S_1$ und $S_2$ bindenden Komponente werden dann sowohl die Konjugate aus an $S_1$ gebundenem Rezeptor und zu bestimmender Substanz sowie an S gebundenem Rezeptor und markierter Verbindung an die Festphase fixiert. Auch hier ist wiederum die Menge an gebundener Markierung ein Maß für die in der Lösung vorliegende Menge an zu bestimmender Substanz. Diese Verfahren weisen eine erhöhte Empfindlichkeit gegenüber bekannten Verfahren auf.

Das erfindungsgemäße Verfahren bietet den Vorteil, daß die Reaktion zwischen Rezeptoren und zu bestimmender Substanz in homogener Phase stattfinden kann, was die Genauigkeit erhöht und die Dauer des Tests erniedrigt.

Die Menge an spezifischem Antikörper bzw. Antigen kann bedeutend verringert werden, da für die Immobilisierung keine spezifisch mit der zu bestimmenden Substanz bindefähigen Rezeptoren erforderlich sind. Dadurch kann der Bedarf an den spezifischen Antikörpern bzw. Antigenen um das 10- bis 20fache erniedrigt werden. Andererseits werden an der Matrix die spezifisch bindenden Substanzen $S_1$ in hohem Überschuß gebunden. Da somit Bindestellen im Überschuß vorliegen, stören unspezifische Anlagerungen bzw. die Bindung endogener Substanzen nicht. Es können somit also Störeinflüsse durch Proben-und Reagenzbestandteile verhindert werden.

Die Erfindung wird noch durch Figuren und Beispiele erläutert.

Fig. 1 zeigt eine Eichkurve für die $T_3$-Bestimmung bei Zugabe von verschiedenen Avidinmengen,

Fig. 2 zeigt eine Eichkurve für die $T_4$-Bestimmung bei Zugabe von verschiedenen Avidinmengen,

Fig. 3 zeigt eine Eichkurve für die $Hb_sAg$-Bestimmung bei Zugabe von verschiedenen Avidinmengen.

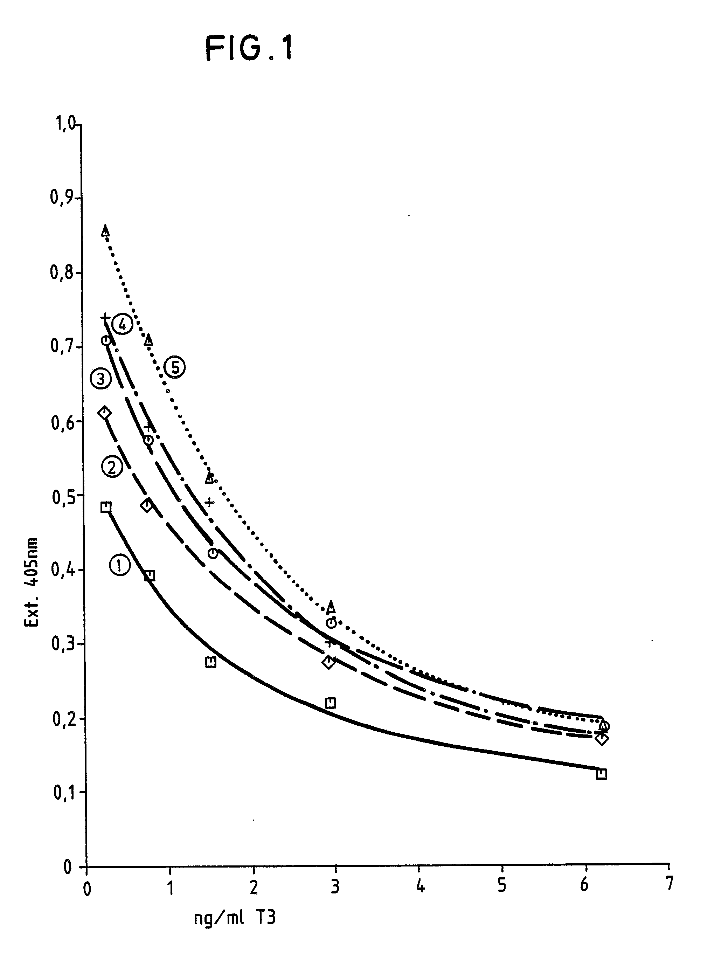

Fig. 1 zeigt die Ergebnisse bei Verwendung von $T_3$-Kontrollösungen, in denen $T_3$ in verschiedener Konzentration eingesetzt wurde, als Probelösungen. Bei den einzelnen Tests wurden verschiedene Avidinmengen eingesetzt, wie der folgenden Aufstellung zu entnehmen ist.

| Kurve 1 | 1 μg Avidin pro tube |
| Kurve 2 | 20 μg Avidin pro tube |
| Kurve 3 | 2 μg Avidin pro tube |
| Kurve 4 | 10 μg Avidin pro tube |
| Kurve 5 | 5 μg Avidin pro tube. |

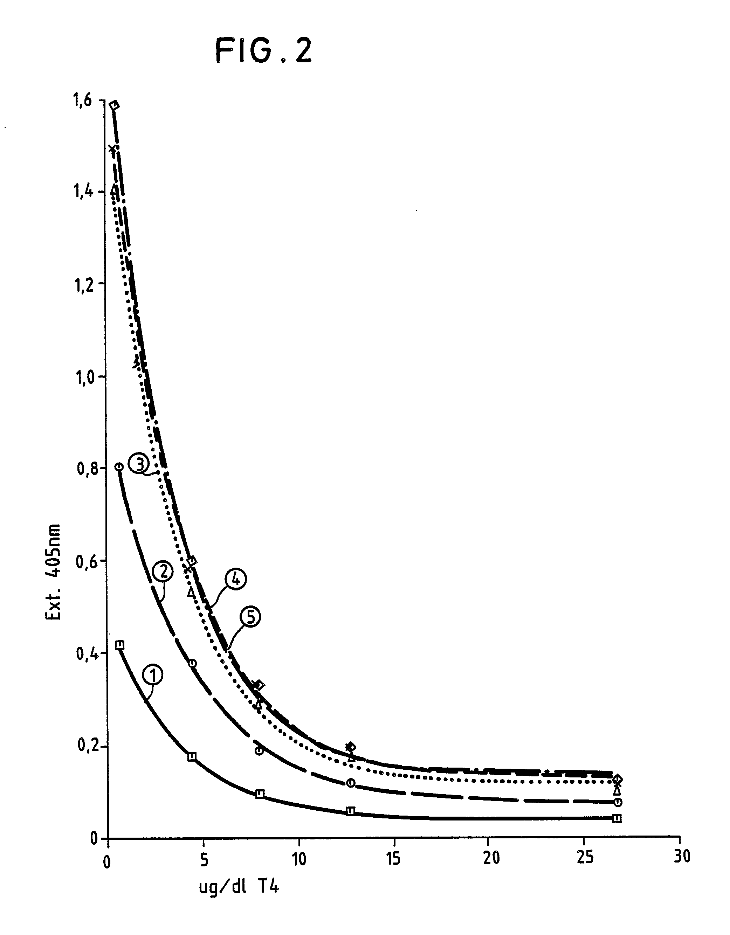

Fig. 2 zeigt eine Eichkurve mit $T_4$-Kontrollösungen, die verschiedene Konzentrationen hatten, sowie die Anwendung unterschiedlicher Avidin-Konzentrationen. Die Avidin-Konzentrationen sind der folgenden Aufstellung zu entnehmen.

| Kurve 1 | 0,5 μg/ml Avidin |
| Kurve 2 | 1,0 μg/ml Avidin |
| Kurve 3 | 2,0 μg/ml Avidin |
| Kurve 4 | 5,0 μg/ml Avidin |
| Kurve 5 | 20,0 μg/ml Avidin. |

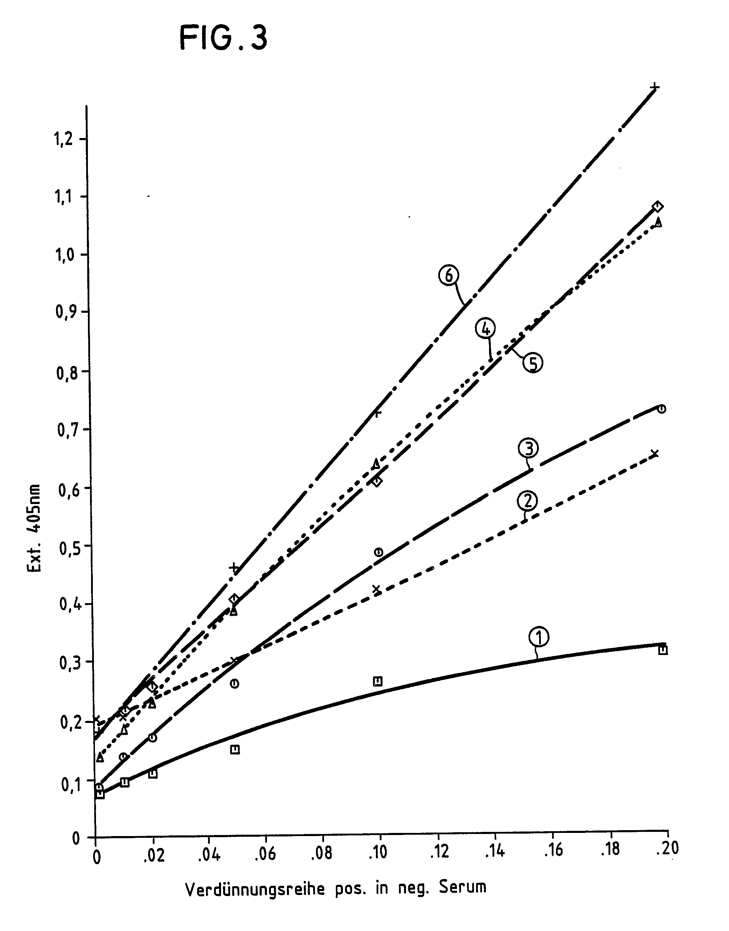

Fig. 3 zeigt eine Eichkurve, die durch regelmäßige Verdünnung eines Serums, das Hepatitis-B-Oberflächenantigen enthielt mit einem Serum, das dieses Antigen nicht enthielt bei verschiedenen Avidin-Konzentrationen erhalten wurde. Die Avidinmengen sind der folgenden Aufstellung zu entnehmen.

| Kurve 1 | 0,5 μg/ml Avidin |
| Kurve 2 | 20,0 μg/ml Avidin |
| Kurve 3 | 1,0 μg/ml Avidin |
| Kurve 4 | 2,0 μg/ml Avidin |
| Kurve 5 | 10,0 μg/ml Avidin |
| Kurve 6 | 5,0 μg/ml Avidin. |

**Beispiel 1**

Herstellung von biotinylierten Polystyroltubes

a) Herstellung von Thermo-RSA (Rinderserumalbumin):

1 g RSA werden in 100 ml 50 mmol/l Kaliumphosphat (pH 7,0) gelöst, auf 70° C erhitzt und 4 Stunden unter leichtem Rühren bei dieser Temperatur gehalten. Die Lösung wird abgekühlt, filtriert und in einer Ultrafiltrationszelle (Ausschlußgrenze: 30.000 Dalton) auf eine Konzentration von 50 mg/ml eingestellt. Anschließend wird gegen das 30fache Volumen an bidest. $H_2O$ dialysiert und anschließend lyophilisiert. Das Produkt hat ein Molekulargewicht von ca. 700.000.

b) Herstellung eines Konjugats aus Biotin und Thermo-RSA:

1140 mg Thermo-RSA-Lyophilisat (ca. 88% Protein) werden in 30 ml $H_2O$ gelöst, mit 5 ml 0,1 mol/l Kaliumphosphatpuffer pH 7,8 auf pH 7,8 eingestellt und danach langsam mit einer Lösung von 122,6 mg Biotin-ε-Aminocarbonsäure-N-Hydroxysuccinimidester (Hersteller Calbiochem) in 2,5 ml Dimethylformamid und 10 ml Dioxan versetzt.
Nach 2 Stunden Rührzeit bei Raumtemperatur wird dreimal 4 Stunden gegen 1 1/2 mmol/l Kaliumphosphatpuffer pH 7,0 dialysiert und anschließend lyophilisiert.

c) Beladung von Polystyrolröhrchen

Zur Beladung wird eine Lösung von
10 μg/ml Konjugat nach Beispiel 1b) in
5,25 g/l Natriumhydrogenphosphat pH 7,4
1,00 g/l Natriumazid verwendet.
In jedes Röhrchen Polystyrol bzw. Luran® (Polystyrol-Acrylnitril-Copolymer, Hersteller BASF) werden 1,5 ml Lösung gefüllt und über Nacht (ca. 22 Stunden) beladen. Danach werden die Röhrchen vollständig entleert und getrocknet.

**Beispiel 2**

Biotinylierung von Antikörpern

Die Biotinylierung von (monoklonalen) Antikörpern wird, wie in J. Histochem. Cytochem. 27, 1131-1139 (1979) beschrieben, durchgeführt. Dazu wird Biotinyl-ε-Aminocarbonsäuren-N-Hydroxysuccinimidester über eine kovalente Peptidbindung an die ε-Aminogruppen der Lysine des Antikörpers gekoppelt.
Dazu wird 1 ml einer Lösung eines über FPLC gereinigten monoklonalen Antikörpers (1 mg/ml), 50 μg einer Lösung von 1 mg/ml Biotinyl-ε-Aminocarbonsäure-N-Hydroxysuccinimidester in DMF zugegeben und 30 Minuten bei Raumtemperatur inkubiert. Anschließend werden die Reaktionsprodukte über eine Ausschlußchromatographie getrennt (Säule: Sephadex G 25 in Entsalzungspuffer). Der Proteingipfel wird ausgiebig gegen phosphat buffered saline mit 0,1% $NaN_3$ dialysiert.

**Beispiel 3**

Verfahren zur Bestimmung von TSH (Sandwichtest)

In ein biotinbeschichtetes Röhrchen (hergestellt nach Beispiel 1) werden 1 ml einer Lösung von 400 ng biotinylierter monoklonaler TSH-Antikörper, hinterlegt bei ECACC unter der internen Nummer I 20 PK 30, in Puffer, bestehend aus
40 mMol/l Natriumhydrogenphosphatpuffer pH 7,0 und
0,5 Gew.-% Detergenz (Pluronic F68)
Konjugat aus monoklonalem TSH-Antikörper, hinterlegt bei ECACC unter der internen Nummer IV 2029 B8, und POD mit einer POD-Aktivität von 75 mU/ml.

Es werden 200 $\mu$l Probe zugegeben und 12 Minuten inkubiert. Danach werden 100 $\mu$l Avidin (5 $\mu$g/ml in obigem Puffer) zugegeben und 2 Stunden bei Raumtemperatur inkubiert. Nach dreimaligem Waschen und Zugabe von 1 ml ABTS®-Substratlösung

100 mMol/l Citratpuffer, pH 4,4
3,2 Mol Perborat
1,9 mMol/l ABTS® (2,2´-Azino-di-(3-ethyl-benzthiazolinsulfonsäure(6))-diammoniumsalz)

wird eine weitere Stunde bei Raumtemperatur inkubiert und anschließend die Extinktion bei 405 nm bestimmt.

## Beispiel 4

Bestimmung von $T_3$ mit kompetitivem Test

Herstellung eines IgG-$T_3$-Polymerisats

30 g Kaninchen-IgG werden in 3 l phosphat buffered saline, pH 8,5, gelöst, 30 Minuten bei Raumtemperatur gerührt und durch Zutropfen innerhalb 15 Minuten mit einer Lösung aus 848 mg N-t-butyloxycarbonyl-$T_3$-Hydroxy-Succinimidester (BOC-$T_3$-Osu) in 200 ml Dioxan versetzt. Nach 3 Stunden bei leichtem Rühren wird die schwachtrübe Lösung filtriert und auf etwa 1/10 des Ausgangsvolumens eingeengt (Endvolumen ca. 300 ml). Zur Entsalzung wird das Konzentrat 16 Stunden gegen vollentsalztes Wasser dialysiert. Das freie BOC-$T_3$-Osu wird mittels Gelfiltration abgetrennt. Man erhält ein Polymerisat aus IgG und $T_3$. Die Biotinylierung dieses Polymerisats erfolgt wie in Beispiel 2 beschrieben.

In ein biotinbeschichtetes Tube werden 500 $\mu$l einer Lösung von 400 ng biotinyliertem $T_3$ Polymerisat in einem Puffer, bestehend aus

120 mMol/l Barbitursäure-Natriumsalz,
18,2 mMol/l Phosphatpuffer, pH 8,6,
0,04 Gew.-% ANS (8-Anilino-1-Naphthalin-Sulfonsäure)
0,2 Gew.-% Rinderserumalbumin und
200 $\mu$l Probe
zugegeben. Nach einer Inkubationszeit von 20 Minuten bei Raumtemperatur wird eine Lösung aus 5 $\mu$g/ml Avidin und einem Konjugat aus POD und einem polyklonalen Antikörper gegen $T_3$ mit einer POD-Aktivität von 200 mU/ml in 500 $\mu$l zugegeben. Nach einer Inkubation über 30 Minuten bei Raumtemperatur wird einmal gewaschen und 1 ml ABTS-Substratlösung (Beispiel 3) zugegeben. Nach 30 Minuten Inkubation bei Raumtemperatur wird die Substratreaktion bei 405 nm verfolgt. Fig. 1 zeigt die Ergebnisse bei Verwendung von $T_3$-Kontrollösungen mit verschiedener Konzentration als Probe bei Zugabe von verschiedenen Avidin-Mengen.

## Beispiel 5

Bestimmung von $T_4$ (kompetitiver Test über biotinylierte Antikörper)

In ein gemäß Beispiel 1 hergestelltes biotinyliertes Polystyrolröhrchen werden 500 $\mu$l eines Reagenzes, bestehend aus

500 ng biotinyliertem polyklonalem Antikörper gegen $T_4$ in einem Puffer, wie im Beispiel 4 beschrieben,

8

bestehend aus:

120 mMol/l Barbitursäure-Natriumsalz

18,2 mMol/l Phosphatpuffer, pH 8,6

0,04 Gew.-% ANS (8-Anilino-1-Naphthalin-Sulfonsäure)

0,2 Gew.-% Rinderserumalbumin

zusammen mit 20 $\mu$l Probe zugegeben. Nach einer Inkubationszeit von 20 Minuten bei Raumtemperatur werden 500 $\mu$l eines weiteren Reagenzes, bestehend aus

200 mU/ml Konjugat aus POD und $T_4$

20 $\mu$g/ml Avidin

120 mMol/l Barbitursäurenatriumsalz

18,2 mMol/l Phosphatpuffer, pH 8,6

zugegeben und 30 Minuten bei Raumtemperatur inkubiert. Nach dreimaligem Waschen mit $H_2O$ werden 1 ml ABTS-Substratlösung (Beispiel 3) zugegeben und 30 Minuten bei Raumtemperatur inkubiert. Anschließend wird die Extinktion bei 405 nm bestimmt. Fig. 2 zeigt eine Eichkurve mit $T_4$-Standards verschiedener Konzentrationen sowie unterschiedlichen Avidinkonzentrationen.

## Beispiel 6

Anti-$Hb_s$ Ag-Test (Antikörperbestimmung)

In ein gemäß Beispiel 1 hergestelltes biotinyliertes Polystyrolröhrchen werden 500 ml eines Reagenzes, bestehend aus

300 ng/ml biotinyliertem $Hb_sAg$ (Herstellung von $Hb_sAg$ und POD-$Hb_sAg$ nach Z. med. Lab. diagn. 28 (1987) 152-156, Biotinylierung wie im Beispiel 2 beschrieben).

120 mU/ml Konjugat aus POD und $Hb_sAg$

40 mMol/l Natriumphosphatpuffer, pH 7,0

0,5 Gew.-% Detergenz (Pluronic F68)

und 200 $\mu$l Probe gegeben. Nach einer Inkubationszeit von 20 Minuten bei Raumtemperatur werden 500 $\mu$l eines Reagenzes, bestehend aus

0,5 $\mu$g/ml - 20 $\mu$g/ml Avidin

zugegeben und 220 Minuten bei Raumtemperatur inkubiert. Nach dreimaligem Waschen mit $H_2O$ und Zugabe von 1 ml ABTS-Substratlösung (Beispiel 3), wird eine Stunde bei Raumtemperatur inkubiert und die Extinktion bei 405 nm bestimmt.

## Ansprüche

1. Verfahren zur Bestimmung einer immunologisch aktiven Substanz nach dem Prinzip des Immunoassays durch Inkubation mit mindestens 2 in flüssiger Phase gelöst vorliegenden Rezeptoren $R_1$ und $R_2$, von denen mindestens einer mit der immunologisch aktiven Substanz bindefähig ist, wobei der zweite Rezeptor $R_2$ eine Markierung trägt, Abtrennung des sich bildenden Komplexes aus der Lösung durch Bindung an eine feste Phase und Messung der Markierung in einer der Phasen,

**dadurch gekennzeichnet,**

daß man einen ersten Rezeptor $R_1$ verwendet, der an eine erste spezifisch bindefähige Substanz $S_1$ gebunden ist, die zu bestimmende immunologisch aktive Substanz mit den Rezeptoren inkubiert in Gegenwart einer Matrix, an die eine zweite spezifisch bindefähige Substanz $S_2$ gebunden ist und daß man nach der Inkubation zur Immobilisierung des Komplexes eine Komponente zugibt, die mindestens je eine spezifische Bindungsstelle für $S_1$ und $S_2$ aufweist und danach die Phasen trennt.

2. Verfahren nach Anspruch 1, **dadurch gekennzeichnet,** daß man eine Matrix verwendet, die aus Kunststoff besteht und daß die zweite spezifisch bindefähige Substanz $S_2$ direkt oder über einen Spacer an die Matrix gebunden wird.

3. Verfahren nach Anspruch 1, **dadurch gekennzeichnet,** daß man die zweite spezifisch bindefähige Substanz $S_2$ an ein hydrophobes Protein mit einem Molekulargewicht von über etwa 500.000 bindet und dieses Protein an der Festphase adsorbiert.

4. Verfahren nach Anspruch 3, **dadurch gekennzeichnet,** daß man als Protein ein Protein mit einem Molekulargewicht von 500.000 bis 20.000.000 verwendet.

5. Verfahren nach Anspruch 3 oder 4, **dadurch gekennzeichnet,** daß man das Protein aus einem löslichen Protein mit einem Molekulargewicht von 10.000 bis 700.000 durch Erhöhung des Molekulargewichts über 500.000 bis 20.000.000 herstellt.

6. Verfahren nach einem der Ansprüche 3 bis 5, **dadurch gekennzeichnet,** daß man das Protein vor der Bindung von $S_2$ mit einem bi- oder polyfunktionellen Proteinreagenz vernetzt, bis das gewünschte Molekulargewicht erreicht ist.

7. Verfahren nach einem der Ansprüche 3 bis 6, **dadurch gekennzeichnet,** daß das Protein vor der Bindung von $S_2$ durch Anwendung von Hitze, Behandlung mit Säuren, denaturierenden Agentien oder chaotropen Ionen und/oder durch chemische Kupplung mit einer hydrophoben Verbindung hydrophobisiert wird.

8. Verfahren nach einem der Ansprüche 3 bis 7, **dadurch gekennzeichnet,** daß man als Protein Rinderserumalbumin, Lipase und/oder Immun-$\gamma$-Globuline verwendet.

9. Verfahren nach einem der vorhergehenden Ansprüche, **dadurch gekennzeichnet,** daß die erste spezifisch bindefähige Substanz $S_1$ und die zweite spezifisch bindefähige Substanz $S_2$ identisch sind und daß die zur Immobilisierung zugegebene Komponente mindestens zwei identische spezifische Bindungsstellen aufweist.

10. Verfahren nach Anspruch 9, **dadurch gekennzeichnet,** daß $S_1$ und $S_2$ Biotin sind und die zur Immobilisierung zugegebene Komponente Avidin oder Streptavidin ist.

11. Verfahren nach einem der vorhergehenden Ansprüche, **dadurch gekennzeichnet,** daß die zu bestimmende immunologisch aktive Substanz ein polyvalentes Antigen, das mindestens zwei Bindungsstellen für Antikörper aufweist oder ein Antikörper ist.

12. Reagenz zur Bestimmung einer immunologisch aktiven Substanz nach dem Prinzip des Immunoassays, enthaltend zwei Rezeptoren $R_1$ und $R_2$, von denen mindestens einer mit der zu bestimmenden immunologisch aktiven Substanz bindefähig ist, wobei $R_2$ eine Markierung trägt, **dadurch gekennzeichnet,** daß an den Rezeptor $R_1$ eine erste spezifisch bindefähige Substanz $S_1$ gebunden ist und daß das Reagenz weiterhin eine Matrix enthält, an der eine zweite spezifisch bindefähige Substanz $S_2$ gebunden ist und daß es physikalisch getrennt davon eine Komponente, die zwei spezifische Bindungsstellen für $S_1$ und $S_2$ aufweist, enthält.

13. Reagenz nach Anspruch 12, **dadurch gekennzeichnet,** daß $S_1$ und $S_2$ Biotin sind und die Komponente Avidin oder Streptavidin ist.

FIG.1

EP 0 322 813 A2

# FIG.2

# FIG.3

Verdünnungsreihe pos. in neg. Serum